# EUROPEAN PATENT SPECIFICATION

(11) **EP 1 985 319 B1**
(45) Date of publication and mention of the grant of the patent: **13.06.2012**
(21) Application number: 07106748.2
(22) Date of filing: 23.04.2007
(51) Int. Cl.: A61L 27/18, A61L 27/34

(54) **Device for cartilage repair**
Vorrichtung zur Knorpelreparatur
Dispositif pour la réparation de cartilages

(43) Date of publication of application: 29.10.2008
(73) Proprietor: Jointsphere B.V., 5611 CZ Eindhoven (NL)
(72) Inventor: Hermsen, Egidius Gerardus Maria, 5611 CZ, Eindhoven (NL); Weinans, Hermannus Hendricus, 3972 AS, Driebergen-Rijsenburg (NL); Versteegen, Ronnij Matthieu, 5963 BA, Hegelsom (NL); Wisse, Eva, 5621 EA, Eindhoven (NL); Meijer, Egbert Willem, 5583 GC, Waalre (NL)
(74) Representative: Brouwer, Hendrik Rogier

(56) References cited:
- WO-A-97/22643
- WO-A-2004/065450
- WO-A-2005/112974
- WO-A2-02/17821
- US-A1- 2005 147 647

## Description

The invention relates to a prosthetic device for use in the joint space between two or more bones, more preferably in the joint space between the femoral condyle and the tibial plateau and/or for use in a bone structure.

Cartilage may be damaged by direct contact injury, inflammation or most commonly, by osteoarthritis. Osteoarthritis is a tissue degeneration process that can accompany daily cartilage wear. In osteoarthritis, damage to the articular surface of joints results from the normal aging process or a traumatic injury, typically resulting from high impact loading in work and/or sports, which progressively worsens over time. The injured cartilage goes through several stages of degradation in which the surface softens, flakes and fragments. Finally, the entire cartilage layer is lost and the underlying subchondral bone is exposed. Cartilage does not possess the capacity to heal easily once damaged. There is therefore a need to provide prostheses having cartilage regenerative properties.

A number of treatments are available to treat articular cartilage damage in joints, such as the knee, starting with the most conservative, non-invasive options and ending with total joint replacement if the damage has spread throughout the joint. Currently available treatments include anti-inflammatory medications in the early stages. Although these may relieve pain, they have limited effect on arthritis symptoms and further do not repair joint tissue. Cartilage repair methods, such as arthroscopic debridement, attempt to at least delay tissue degeneration. These methods however are only partly effective at repairing soft tissue, and do not restore joint spacing or improve joint stability. Joint replacement (arthroplasty) is considered as a final solution, when all other options to relieve pain and restore mobility have failed or are no longer effective. While joint arthroplasty may be effective, the procedure is extremely invasive, technically challenging and may compromise future treatment options. Cartilage regeneration has also been attempted, more in particular by tissue-engineering technology. The use of cells, genes and growth factors combined with scaffolds plays a fundamental role in the regeneration of functional and viable articular cartilage. All of these approaches are based on stimulating the body's normal healing or repair processes at a cellular level. Many of these compounds are delivered on a variety of carriers or matrices including woven polylactic acid based polymers or collagen fibers. Despite various attempts to regenerate cartilage using arthroscopic techniques, such as for instance drilling of holes to promote cell infiltration from the bone marrow, a reliable and proven treatment does not currently exist for repairing defects to the articular cartilage.

Because the cartilage layer lacks nerve fibers, patients are often not aware of the severity of the damage. During the final stage, an affected joint consists of bone rubbing against bone, which leads to severe pain and limited mobility. By the time patients seek medical treatment, surgical intervention may be required to alleviate pain and repair the cartilage damage. Prostheses have been developed for the joint in order to avoid or postpone such surgical interventions. These are often implanted in an early stage of damage, and are provided for preventive treatment, in order to avoid unnoticed degeneration of the joint.

A known prosthesis is described in U. S. Patent No. 5,171,322. Herein a biocompatible, well deformable, flexible, resilient material is described that is placed in the meniscus and attached to soft tissue surrounding the knee joint. However, the known prosthesis has not been able to achieve the load distribution properties similar to a human meniscus, and moreover does not help in regenerating possibly damaged cartilage.

WO 2004/065450 A2 discloses an implant for use in a living organism and comprises a biodegradable polyurethane composition including hard and soft segments, and a bioactive agent incorporated therein. The bioactive agent is incorporated into the polyurethane by reacting isocyanate groups of at least one multifunctional isocyanate compound with the bioactive agent, which has to have at least one hydroxyl or amine group. To release the bioactive agent, the polyurethane compositions must degrade into biocompatible degradation products, including the bioactive agent.

WO 2005/112974 A2 discloses peptide-modified polyurethanes. The peptide is incorporated into the backbone chain of the polyurethane polymer.

WO 02/17821 A2 discloses implantable material for resurfacing that can be a polyurethane having hard and soft segment domains. Although bioactivity is attached, WO 02/17821 A2 is silent about how this is achieved.

It is therefore an object of the invention to provide a prosthetic device having improved load distribution as well as cartilage regenerating properties.

The prosthetic device according to the invention thereto comprises a body at least partly formed from a biocompatible elastomer, in particular a segmented thermoplastic elastomer having crystallized blocks, and at least one functional component, which is able to chemically bond to the crystallized blocks, and has cartilage regenerative properties, wherein the crystallized blocks comprise bis-urea moieities. The use of a segmented thermoplastic elastomer (hereinafter also referred to as TPE), instead of a chemically crosslinked rubber allows to mould the prosthetic device into the right shape that is individual to the patient. This can for instance be carried out by heating, since in TPE, the crosslinks can be broken reversibly as they are of a physical nature. TPE are polymers that combine advantages of both thermoplastic polymers and elastomers. The specific properties of TPE are a result of their morphology. At ambient temperature, the physical crosslinks in the amorphous matrix give the material its elastomeric, rubber-like properties. At higher temperatures, these physical crosslinks are broken (reversibily), and the material can be processed easily, characteristic for thermoplastic. The TPE according to the invention are segmented copolymers, where the reversible physical crosslinks originate from crystallization of one of the blocks of the segmented copolymer. Particularly preferred TPE contain 'hard' crystallized blocks of polyester, polyamide, and/or polyurethane segments. TPE are used in the prosthetic device of the invention since they combine mechanical stability at low temperatures, i.e. at body temperature, and easy processability and formability at higher temperatures, more in particular at temperatures above the melting point of the hard blocks.

A preferred embodiment of the prosthetic device according to the invention is characterized in that the segmented thermoplastic elastomer is a thermoplastic elastomeric polyurethane (TPU). The TPU comprises basically three building blocks: a long-chain diol, for example with a polyether or polyester backbone, a diisocyanate, and, finally, a chain extender, such as water, a short-chain diol, or a diamine. TPU are typically prepared in a one pot procedure, in which the long-chain diol is first reacted with an excess of the diisocyanate, to form an isocyanate functionalized prepolymer. The latter is subsequently reacted with the chain extender which results in the formation of the high molecular weight polyurethane. If a diamine is used as the chain extender, the TPU will also contain urea moieties, which is preferred. At room temperature, the low melting soft blocks are incompatible with the high melting hard blocks, which induces microphase separation by crystallization or liquid-liquid demixing.

The synthetic procedure to prepare TPU generally leads to a distribution in the hard block lengths. As a result, the phase separation of these block copolymers is incomplete. Part of the hard blocks, in particular the shorter ones, are dissolved in the soft phase, causing an increase in the glass transition temperature, which is undesired for the low temperature flexibility and elasticity of the material. The polydisperse hard block is manifested in a broad melting range and a rubbery plateau in dynamic mechanical thermal analysis (DMTA that is dependent on temperature, i.e. is not completely flat. In order to solve this problem, preferably block copolymers containing hard blocks of substantially uniform length are used in the prosthetic device. Preferred examples of types of hard blocks include non-hydrogen bonding polyurethane moieties, polyurethane urea moeities, and aramid moeities. TPE containing substantially uniform hard blocks may be prepared by fractionation of a mixture of hard block oligomers, and subsequent copolymerization of the uniform hard oligomer of a specific length with the prepolymer.

The prosthesis comprises a segmented TPE with crystallized blocks comprising bis-urea moieties. TPE with hard blocks based on, preferably uniform, bis-urea moieties have the advantage that their synthesis makes use of simple isocyanate chemistry. These TPE may for instance be prepared by a chain extension reaction of an isocyanate functionalized prepolymer with a diamine, or by a chain extension reaction of an amine functionalized prepolymer with a diisocyanate. Examples of suitable, commercially available diamines and diisocyanates include alkylene diamines, diisocyanates, arylene diamines and/or diisocyanates. Amine functionalized prepolymers are also commercially available, or can be prepared from (readily available) hydroxy functionalized prepolymers by cyanoethylation followed by reduction of the cyano-groups, by Gabriel synthesis (halogenation or tosylation followed by modification with phthalimide, and finally formation of the primary amine by deprotection of the phthalimide group) or by other methods that are known in the art. Isocyanate functionalized prepolymers can be prepared by reaction of hydroxy functionalized prepolymers with diisocyanates, such as for example isophorone diisocyanate (IPDI), 1,4-diisocyanato butane, 1,6-diisocyanato hexane or 4,4'-methylene bis(phenyl isocyanate). Alternatively, isocyanate functionalized prepolymers can be prepared from amine functionalized prepolymers, for example by reaction with di-tert-butyl tricarbonate. Hydroxy functionalized prepolymers of molecular weights typically ranging from about 500 g/mol to about 5000 g/mol of all sorts of compositions are also advantageously used. Examples include prepolymers of polyethers, such as polyethylene glycols, polypropylene glycols, poly(ethylene-co-propylene) glycols and poly(tetrahydrofuran), polyesters, such as poly(caprolactone)s or polyadipates, polycarbonates, polyolefins, hydrogenated polyolefins such as poly(ethylene-butylene)s, etc.

According to the invention, the prosthetic device comprises a body at least partly formed from a biocompatible elastomer, which includes at least one functional component, able to chemically bond to the crystallized blocks, and having cartilage regenerative properties. A particularly preferred functional component comprises a peptide, even more preferred a peptide comprising at least one RGD-sequence, and most preferred a peptide comprising a RGD sequence capable of binding integrins and thereby stimulating cell adhesion; and/or comprising a RGD sequence with specific flanking amino acids such that it contains motifs from extracellular cartilage matrix molecules, such as fibronectin, COMP and/or others. These peptides not only stimulate cell adhesion but preferably also induce proper chondrocyte differentiation such that the synthesis of collagen type 2 may increase and collagen type 1 may decrease. In addition molecules that induce catabolic effects on the cartilage such as MMPs, ILs and/or TNFs may decrease as well. The peptide sequence is preferably fine tuned such that the newly synthesized cartilage will have optimal mechanical properties that mimic the host cartilage. Peptides comprising at least one RGD-sequence are known per se, but not in the particular combination with the TPE and/or prosthetic device of the invention. In order to incorporate the functional component into the TPE, several possibilities exist. A particularly preferred TPE having at least one functional component comprises uniform bis-urea moieties. TPEs with hard blocks that are based on uniform bis-urea units have an additional advantage due to the presence of these bisurea units and due to the specific morphology of these TPEs. The bis-urea units in the polymer chains stack via (reversible) hydrogen bonding interactions to form the phase separated hard blocks. Due to the uniformity and specific length between the ureas, the bis-urea structural element can be employed as a recognition site for the reversible binding of guest molecules. Functionality can be introduced into the bis-urea stack and therefore into the polymer material. This is achieved by adding a functional component, for example a dye or a peptide, that preferably also bears the specific bis-urea group, for instance a functionality that bears a bis-ureido-butylene moiety is incorporated into the bis-ureido-butylene stack of a TPE, and is thereby anchored into the polymer material.

According to the present invention, related to a prosthetic device for the human body, it is particularly preferred that peptides with a certain specific function (promotion of cell binding, promotion of cell growth, etc.) are modularly added to the TPE of choice. Thereby a biofunctional, and biocompatible material may be obtained. Particularly preferred is a prosthesis, wherein the at least one functional component comprises a peptide. Even more preferred is a prosthesis, wherein the peptide comprises at least one RGD-sequence. Most preferred is a peptide comprising a RGD sequence capable of binding integrins and thereby stimulating cell adhesion; and/or comprising a RGD sequence with specific flanking amino acids such that it contains motifs from extracellular cartilage matrix molecules, such as fibronectin, COMP and/or others. These peptides not only stimulate cell adhesion but preferably also induce proper chondrocyte differentiation such that the synthesis of collagen type 2 may increase and collagen type 1 may decrease. In addition molecules that induce catabolic effects on the cartilage such as MMPs, ILs and/or TNFs may decrease as well. The peptide sequence is preferably fine tuned such that the newly synthesized cartilage will have optimal mechanical properties that mimic the host cartilage. This readily promotes growth of cartilage cells of hyaline type, which results in strong and wear resistant cartilage.

The prosthetic device according to the invention can deform to distribute the physiologic loads over a large area such that the joint space is maintained under physiologic loads. The body of the prosthesis preferably has a shape that is contoured to fit with the femoral condyle, the tubercle, and the tibial plateau but is allowed to translate within the joint space. As is well known by those skilled in the art, the femoral condyle, tubercle, and tibial plateau of a given knee may vary in shape and size. As such, while various specific shapes are shown and described herein, it should be understood that various other shapes and configurations are within the scope of the present invention. Moreover, the prosthetic device is preferably used without any means of attachment and remains in the joint space by its geometry and the surrounding soft tissue structures. The prosthesis can also be used for other joint spaces, such as a temporal-mandibular joint, an ankle, a hip, a shoulder, for instance. The use of the segmented elastomer in the prosthesis of the invention yields a compliant, wear-resistant prosthesis, having load distribution capabilities similar to native articular cartilage and meniscus.

In another preferred embodiment of the prosthesis according to the invention, the body further comprises a reinforcing material selected from the group consisting of polymers and/or metals. In an even more preferred embodiment, the reinforcing material is a foam, preferably a metal foam. In a particularly preferred embodiment the foam forms the core of the body and the elastomer the skin. This embodiment allows bone ingrowth into the foam, whereby a strong fixation is build between prosthesis and bone. Cartilage cells from the host cartilage having a strong affinity for the segmented elastomer skin of body, will colonise the surface thereof and will be triggered by the polymer with its peptides to produce new hyaline cartilage tissue.

In a preferred embodiment of the prosthesis according to the invention the RGD sequence containing peptides, optionally having flanking amino acids, should stand out over some distance from the surface of the segmented thermoplastic elastomer to further the cell adhesive properties. This can be achieved for instance by adding Glycines to the peptide. The spacer preferably has a length in the order of 2 - 30 glycine molecules (corresponding to about 7 - 100 angstrom). The surface density of the active molecule that is binded to the elastomer can become active for values as low as 10fmol/cm² but is preferably in the order of 1-10 pmol/cm² to have optimal binding and regulatory effects. To prevent the functional component from negatively affecting the (mechanical) properties of the elastomer, the preferred amount of the functional component, which preferably is a peptide, with respect to the total amount of bis-urea moieties in the elastomer, is lower than 50 mol%, more preferably lower than 30 mol%, and most preferably lower than 20 mol%.

The invention will know be further elucidated by the following figures and examples, without however being limited thereto. In the figures:
Figure 1 depicts a schematic view from above of an exemplary medial meniscus prosthesis according to the present invention;
Figure 2 depicts a schematic side view along the line AA' of the embodiment shown in figure 1;
Figure 3 depicts a schematic side view along the line BB' of the embodiment shown in figure 1;
Figure 4 depicts a schematic representation of a TPU and its building blocks; in this case the chain extender is a short diol;
Figure 5 depicts a schematic representation of the morphology of the segmented copolymer TPE according to the present invention.

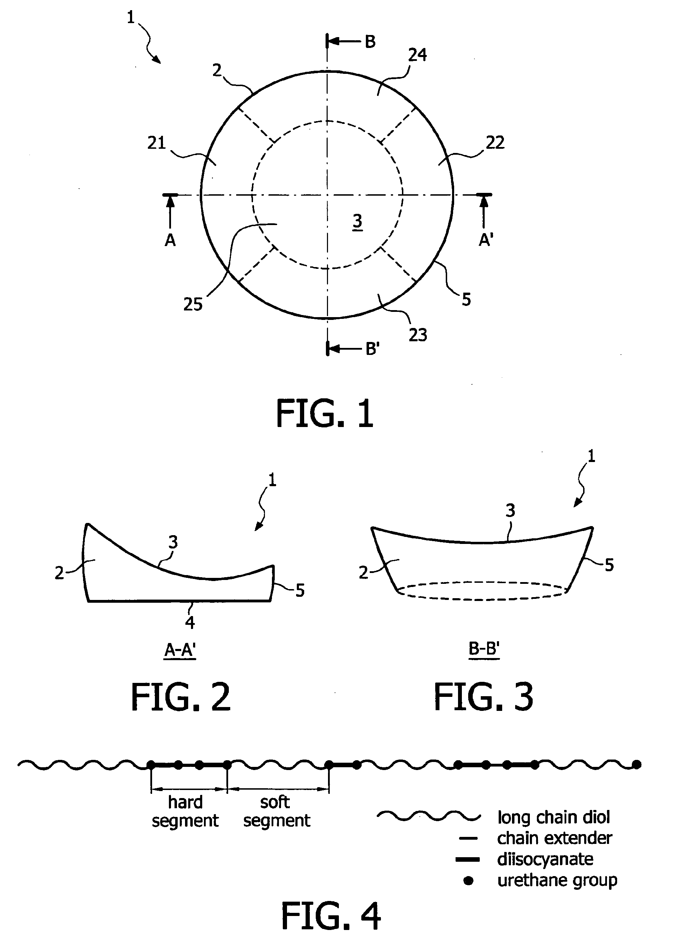

Referring to figures 1, 2 and 3, a prosthesis 1, generally elliptical in shape, comprising a body 2 formed from a segmented copolymer TPE is shown. In a preferred embodiment, the prosthesis 1 is kidney shaped, but other shapes may be used as well. In particular, the body 2 may be toroidal, circular, planar, donut shaped or crescent shaped. The prosthesis 1 is intended for use in a medial compartment of a knee. It should be understood that a device according to the present invention may have a shape being the mirror image of the device illustrated in figure 1, depending on which knee is contemplated. The body 2 of the prosthesis 1 has a superior surface 3, an inferior surface 4, and an outer wall 5. The superior surface 3 generally forms a concave surface, contoured to fit with a femoral condyle while the inferior surface 4 forms a generally convex surface, contoured to fit on top of a tibial plateau. As shown in figure 3, the inferior surface 4 may also be shaped concavely. The body 2 further includes a cruciate region 21, an outer region 22, an anterior region 23, a posterior region 24 and a central region 25. The outer wall 5 is formed from the periphery of the cruciate region 21, outer region 22, anterior region 23, and posterior region 24. The various regions are contiguous but may not be clearly delineated. Instead, the regions are defined merely to provide a point of reference for various aspects of the present invention. The prosthesis 1 is wide enough to fully receive the width of the femoral condyle. The length of the prosthesis 1 is approximately equal to the anterior-posterior length of the tibial plateau. By being wider, the prosthesis 1 is able to provide a channel to guide the femoral condyle, aiding the prosthesis 1 to maintain its position within the space between two bones ("joint space") during kinematic joint motion of the knee. By simultaneously having a preferably convex inferior surface 4, contoured to receive the tibial plateau, the prosthesis 1 maintains its position within the joint space. While a secure fit within the joint space is needed, it should be understood that the prosthesis 1 may shift slightly or translate during movement of the joint. In relation to the knee joint, the prosthesis 1 must for instance be able to engage in natural motion, including flexion and extension motions commonly associated with typical movement, without unrecoverably unseating from the tibial plateau. As used herein, "unrecoverably unseating" refers to a shift in the positioning of the device that is so significant that it is unable to return to its original position. As is clear from figure 3, the posterior region 24 has a slightly greater thickness than the anterior region 23. The greater thickness of the prosthesis 1 at its posterior region 24 aids the prosthesis 1 to stay in place by forming a barrier to anterior displacement through the joint space. The greater thickness of the posterior region 24, however, does not pose a problem during insertion due to the compliant nature of the segmented elastomer. Generally the thickness of the posterior region 24 ranges between about 2 and 15 mm while the anterior region 23 ranges between 1 and 12 mm. The cruciate region 21, the outer region 22, and the central region 25 may have thicknesses ranging from 1 to 20 mm. A prosthesis 1 according to the present invention may include one or more sloped areas in the various regions and surfaces to enable the prosthesis 1 to stay on the tibial plateau during flexion and extension without the need for any additional securing means. Specifically, the geometry of the prosthesis 1 is selected to enable the body 2 to fit between the tibial plateau and the femoral condyle while taking into account the tubercle without the need for cement, pinning, or other surgical securement means. While preferably the prosthesis 1 does not require a means of attachment beyond its geometry, a tissue fixation component, such as tabs or holes to allow the surgeon to suture the prosthesis 1 to native body structures, may be combined with the prosthesis 1 to enhance tissue fixation.

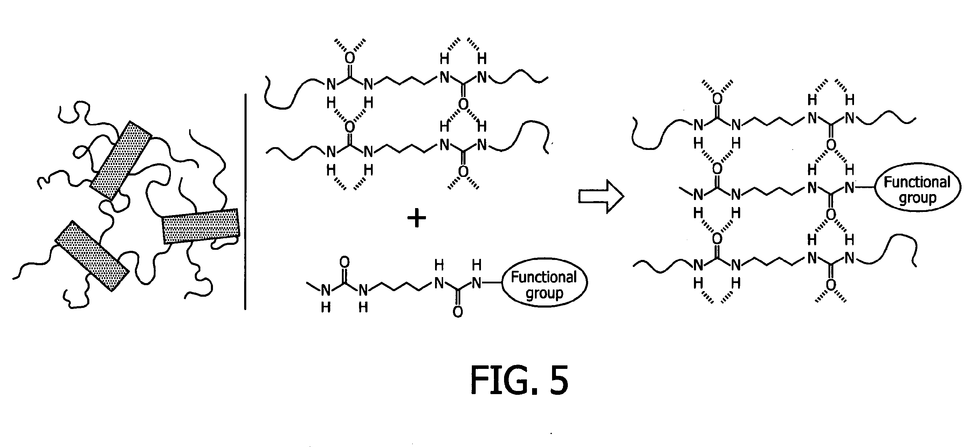

According to the invention the prosthesis is made of segmented thermoplastic elastomer having crystallized blocks, and at least one functional component, which is able to chemically bond to the crystallized blocks, and has cartilage regenerative properties, wherein the crystallized blocks comprise bis-urea moieities. An example of a preferred TPU and its building blocks is shown in figure 4. In the embodiment shown the chain extender is a short diol. In figure 5 a schematic representation of the morphology of the segmented copolymer TPE according to the present invention is shown. The depicted TPE with hard blocks based on uniform bis-urea units have the desired properties due to the presence of said bisurea units and due to the specific morphology of these TPE. Referring to figure 5 (left side) the bis-urea units in the polymer chains stack via (reversible) hydrogen bonding interactions to form the phase separated hard blocks. Due to the uniformity and specific length between the ureas, the bis-urea structural element can be employed as a recognition site for the reversible binding of guest molecules. Functionality can be introduced into the bis-urea stack and therefore into the segmented elastomer. This is achieved by adding a peptide, that also bears the specific bis-urea group. This is shown in figure 5 on the right: a functionality that bears a bis-ureido-butylene moiety is incorporated into the bis-ureido-butylene stack of the TPE, and is thereby anchored into the elastomer. According to the invention, peptides with a certain specific function (promotion of cell binding, promotion of cell growth, etc.) can be modularly added to the TPE, thereby making a biofunctional material. A particularly preferred prosthesis comprises a peptide comprising at least one RGD-sequence. Most preferred is a peptide comprising a RGD sequence capable of binding integrins and thereby stimulating cell adhesion; and/or comprising a RGD sequence with specific flanking amino acids such that it contains motifs from extracellular cartilage matrix molecules, such as fibronectin, COMP and/or others.

### Examples 1 to 19

### Materials used

Bis(3-aminopropyl)-poly(tetrahydrofuran) with molecular weight 1100 g/mol and hydroxy terminated poly(tetrahydrofuran) with molecular weight 2000 g/mol were purchased from Aldrich. Hydroxy terminated rando m copolymer of THF (tetrahydrofuran) and EO (ethylene oxide) of molecular weight 4000 g/mol was kindly provided by Akzo-Nobel (ca. 10% of the monomeric units are EO), and hydroxy-terminated poly(ethylene-ran-butylene) (hydrogenated polybutadiene, Kraton liquid polymer L-2203, Mn = 3500 g/mol) was kindly provided by Kraton Polymers Research. 1,4-Diisocyanatobutane, 1,3-phenylenediisocyanate, 4,4'-methylenebis(phenylene diisocyanate), borane-tetrahydrofuran complex (1 M in THF), and sodium hydride (60% dispersion in mineral oil) were purchased from Aldrich. 1,2-Ethylenediamine was purchased from Acros. 1,6-Diisocyanatohexane was purchased from Fluka. di-tert-Butyl tricarbonate was prepared according to literature proceedings (Peerlings, H.W.I. and Meijer, E.W., Tetrahedron Letters, 1999, 40, 1021), as well as N-carbobenzoxy-6-aminohexanoic acid (Shah, J. et al., J.Med.Chem., 1995, 38, 4284). Poly(e-caprolactone)diol (Mn = 1250 and 2000 g/mol), dicyclohexylcarbodiimide (DCC), p-toluenesulphonic acid·H2O and 4-(N,N'-dimethyl)aminopyridine (DMAP) were purchased from Acros. Sodium hydroxide (NaOH), 4 Å molsieves and Pd/C(10%) were purchased from Merck. Dibutyltin dilaurate, 1,4-diaminobutane and hexylamine were purchased from Aldrich. Sodium dodecyl sulfate (SDS), 1-hydroxybenzotriazole hydrate (HOBt), diisopropylcarbodiimine (DIPCDI) and 6-(Fmoc-amino)caproic acid were purchased from Fluka. Wang-resin (D-1250) loaded with 0.63 mmol gram-1 FMOC protected serine (FMOC-Ser(tBu)), FMOC-Asp(OtBu), FMOC-Glycine and FMOC-Arg(PMC) were purchased from Bachem. All solvents were purchased from Biosolve. Deuterated solvents were purchased from Cambridge Isotope Laboratories. Water was always demineralized prior to use. Chloroform was dried over molsieves. Further chemicals were used without further purification. All reactions were carried out under a dry argon atmosphere, except for the synthesis of the peptide.

### Equipment used

Infra red spectra were measured on a Perkin Elmer Spectrum One FT-IR spectrometer with a Universal ATR Sampling Accessory. 1H-NMR and 13C-NMR spectra were recorded on a Varian Gemini 300 MHz or a Varian Mercury 400 MHz NMR spectrometer. Molecular weights of the synthesized polycaprolactone polymers were determined by size exclusion chromatography (SEC) using a poly(styrene) calibrated PL-SEC 120 high temperature chromatograph that was equipped with a PL gel 5µm mixed-C column, an autosampler and an RI detector at 80 oC in 1-methyl-2-pyrrolidinone (NMP). The poly(tetrahydrofuran) polymers were analyzed with SEC on a Shimadzu LC10-AT, using a Polymer Laboratories Plgel 5µm mixed-D column, a Shimadzu SPD-10AV UV-Vis or a Shimadzu RID-6S detector, and NMP as eluent; polystyrene standards were used for calibration. Differential Scanning Calorimetry (DSC) measurements were performed on a Perkin Elmer Differential Scanning Calorimeter Pyris 1 with Pyris 1 DSC autosampler and Perkin Elmer CCA7 cooling element under a nitrogen atmosphere. Melting and crystallization temperatures were determined in the second heating run at a heating/cooling rate of 10 oC min-1, glass transition temperatures at a heating rate of 40 oC min-1. Optical properties and flow temperatures were determined using a Jeneval polarization microscope equipped with a Linkam THMS 600 heating device with crossed polarizers. MALDI-TOF spectra were obtained on a Perseptive Biosystems Voyager DE-Pro MALDI-TOF mass spectrometer (accelerating voltage: 20kV; grid voltage: 74.0%, guide wire voltage: 0.030%, delay: 200 ms, low mass gate 900 amu). Samples for MALDI-TOF were prepared by adding a solution of the polymers in THF (20 ml, c=1 mg/ml) to a solution of a-cyano-4-hydroxycinnamic acid in THF (10 ml, c=20 mg/ml) and subsequent thoroughly mixing. This mixture (0.3 ml) was brought on a sample plate, and the solvent was evaporated. Reversed phase liquid chromatography - mass spectroscopy (RPLC-MS) was performed on a system consisting of the following components: Shimadzu SCL-10A VP system controller with Shimadzu LC-10AD VP liquid chromatography pumps with an Alltima C18 3u (50 mm x 2.1 mm) reversed phase column and gradients of water-acetonitrile-isopropanol (1:1:1 v/v supplemented with 0.1% formic acid), a Shimadzu DGU-14A degasser, a Thermo Finnigan surveyor autosampler, a Thermo Finnigan surveyor PDA detector and a Finnigan LCQ Deca XP Max.

### Example 1: [pTHF1100-U-C4H8-U]n, with U representing a urea group.

Bis(3-aminopropyl)-poly(tetrahydrofuran), Mn=1100 g/mol, (10.00 g, 9.09 mmol) was dissolved in chloroform (100 ml), and to this solution a solution of 1,4-diisocyanatobutane (1.4 g, 9.99 mmol) in chloroform (40 ml) was added dropwise. The mixture was stirred for 1 h, and subsequently partly concentrated, and methanol (5 ml) was added. The product was precipitated in hexane (500 ml), filtered and dried in vacuo. It was obtained as white, fluffy, elastic fibers (10.62 g, 93%). 1H-NMR (CDCl3): d 5.4-4.8 (4H, NH), 3.41 (58H, CH2O), 3.25 (4H, OCH2CH2CH2N), 3.17 (4H, NCH2CH2CH2CH2N), 1.74 (4H, OCH2CH2CH2N), 1.62 (58H, OCH2CH2CH2CH2O), 1.50 (4H, NCH2CH2CH2CH2N). FT-IR (ATR): n 3324 (N-H stretching), 2940, 2854, 1615 (C=O stretching), 1580 1365, 1104 (C-O stretching) cm-1. SEC (NMP, rel. to PS): Mn = 42*103 g/mol. DSC: Tg = -68 °C, Tm = 102 °C. T-flow = 140 °C.

### Example 2: [pTHF1100-U-X-U]n, with X = n-C6H12, metha-Ph or para-(Ph-CH2-Ph)

In a similar way as in Example 1, bis(3-aminopropyl)-poly(tetrahydrofuran) Mn=1100 g/mol was reacted at room temperature with 1 molar equivalent of 1,6-diisocyanatohexane (X = n-C6H12), 1,3-phenylenediisocyanate (X = metha-Ph) or 4,4'-methylenebis(phenyl isocyanate) (X = para-(Ph-CH2-Ph)), using chloroform as reaction solvent. After isolation by precipitation and drying, the polymer products had molecular weights of Mn = 43*103 g/mol (X = n-C6H12), 38*103 g/mol (X = metha-Ph) and 55*103 g/mol (para-(Ph-CH2-Ph)) as measured with SEC using NMP as eluent and relative to polystyrene standards. The isolated polymers were all three obtained as highly elastic fluffy materials, and could be solvent casted from chloroform to obtain a transparent elastic film after evaporation of the solvent.

### Example 3: [pTHF1100-U-C2H4-U]n

Bis(3-aminopropyl)-poly(tetrahydrofuran), Mn=1100 g/mol, (0.50 g, 0.45 mmol) was dissolved in chloroform (10 ml), and a solution of di-tert-butyl tricarbonate (0.235 g, 0.91 mmol) in chloroform (1 ml) was injected into this solution. The reaction mixture was stirred for 30 min. during which time the amines were converted to isocyanate groups. Then, 1,2-ethylenediamine (0.0269 g, 0.45 mmol) in chloroform (3 ml) was added dropwise, and the solution was stirred for 1 h, and subsequently partly concentrated, and methanol (1 ml) was added. The product was precipitated in pentane (50 ml), filtered and dried in vacuo. The product was obtained as white, fluffy, elastic fibers (0.47 g, 86%). 1H-NMR (DMSO): d 5.91 (4H, NH), 3.34 (59H, CH2O), 2.99 (8H, CH2N), 1.51 (56H, CH2CH2CH2). FT-IR (ATR): n 3329 (N-H stretching), 2937, 2854, 1615 (C=O stretching), 1589 1366, 1105 (C-O stretching) cm-1. SEC (NMP, rel. to PS): Mn=41*103 g/mol. T-flow = 115 °C.

### Example 4: Bis(2-cyanoethyl)-poly(tetrahydrofuran), Mn = 2000 g/mol

Poly(tetrahydrofuran) diol, Mn=2000 g/mol, (20.00 g; 10.0 mmol) and 15-crown-5 (44 mg; 0.2 mmol) were dissolved in acrylonitrile (40 ml) and cooled on an icebath. Sodium hydride (8 mg 60% dispersion in mineral oil; 0.2 mmol) is added to the solution, and the reaction mixture is stirred at 0°C for about 15 min, after which the reaction mixture turned slightly yellow. At this point, the reaction was quenched by addition of a drop of concentrated hydrochloric acid. The solution was concentrated, the residue taken up in dichloromethane (100 ml) and centrifuged at 4500 rpm. The mixture was decanted, filtered, and concentrated in vacuo. The product was obtained as a slightly yellow, viscous liquid, that slowly crystallized (20.13 g, 96%). 1H-NMR (CDCl3): d 3.62 (t, 4H, OCH2CH2CN), 3.51 (t, 4H, CH2OCH2CH2CN), 3.40 (br. t, 106H, OCH2CH2CH2CH2O main chain), 2.59 (t, 4H, CH2CN), 1.60 (br. m, 110H, OCH2CH2CH2CH2O main chain). 13C-NMR (CDCl3): d 117.7 (CN), 71.0 (CH2OCH2CH2CN), 70.4 (OCH2CH2CH2CH2O main chain), 65.1 (OCH2CH2CN), 26.3 (OCH2CH2CH2CH2O main chain), 18.7 (CH2CN). FT-IR (ATR): n 2939, 2855, 2161 (w, C°N stretching), 1367, 1103 (C-O stretching) cm-1.

### Example 5: Bis(3-aminopropyl)-poly(tetrahydrofuran), Mn = 2000 g/mol

To a solution of borane-tetrahydrofuran complex (80 ml 1M in THF, 80 mmol) in dry THF (240 ml) was added slowly bis(2-cyanoethyl)-poly(tetrahydrofuran) of Example 4 (20.00 g, 9.5 mmol) dissolved in dry THF (160 ml) at 0°C. The solution was stirred for 30 min at 0°C, after which it was heated to reflux for 4 h. The reaction mixture was cooled to 0°C, and methanol (80 ml) was added dropwise. (Be careful: hydrogen-gas evolution). Hydrochloric acid (4 ml, 37% in water) was added slowly, and the reaction mixture was stirred for 1 h, and subsequently evaporated to dryness under reduced pressure. Trimethyl borate was removed by three coevaporations with methanol (3 times 100 ml). To the viscous liquid was added sodium hydroxide solution (150 ml, 1M in water), and this was extracted with diethyl ether (3 times 300 ml). The combined organic layers were dried with sodium sulfate, filtered, and the solvent was evaporated on a rotary evaporator without putting the flask in the water bath. During the evaporation, the polymer precipitated from the cold solution and was obtained as a white powder (18.74 g, 93%). 1H-NMR (CDCl3): d 3.49 (t, 4H, OCH2CH2CH2NH2), 3.41 (br. t, 138H, OCH2CH2CH2CH2O main chain), 2.79 (t, 4H, CH2NH2), 1.71 (t, 4H, OCH2CH2CH2NH2), 1.62 (br. m, 142H, OCH2CH2CH2CH2O main chain), 1.1 (br. s, 4H, NH2). 13C-NMR (CDCl3): d 70.5 (OCH2CH2CH2CH2O main chain), 68.8 (OCH2CH2CH2NH2), 39.7 (CH2NH2), 33.6 (OCH2CH2CH2NH2), 26.4 (OCH2CH2CH2CH2O main chain). FT-IR (ATR): n 3564, 3539, 2941, 2862, 1492, 1372, 1107, 996 cm-1. MALDI-TOF [M+Na+] = Calcd. 155.1 + n*72.0 Da. Obsd. 155.9 + n*72.0 Da. SEC (phenyl urea derivative): Mn=3769 g/mol, PDI=1.5. Mn according to 1H NMR: 2500 g/mol.

### Example 6: [pTHF2000-U-C4H8-U]n

In a similar way as described in Example 1 for bis(3-aminopropyl)-poly(tetrahydrofuran) Mn=1100 g/mol, bis(3-aminopropyl)-poly(tetrahydrofuran) Mn=2000 g/mol from Example 5 was reacted at room temperature with 1 molar equivalent of 1,4-diisocyanatobutane using chloroform as reaction solvent. After similar work-up as described in Example 1, the isolated polymer product was obtained as a white elastic fluffy material. The polymer product had a molecular weight of Mn = 53*103 g/mol as measured with SEC using NMP as eluent and relative to polystyrene standards. DSC: Tg = -74 °C, Tm1 = 1 °C, Tm2 = 101 °C. T-flow = 125 °C.

### Example 7: Bis(3-aminopropyl)-poly(tetrahydrofuran-ran-ethyleneoxide), Mn = 4000 g/mol

In a similar way as described in examples 4 and 5 for hydroxy terminated poly(terahydrofuran) Mn = 2000 g/mol, hydroxy terminated poly(tetrahydrofuran-ran-ethylene oxide) Mn = 4000 g/mol was transformed to its bis(3-aminopropyl) analogue. Briefly, first the hydroxy terminated polymer was cyanoethylated, and then the resulting cyano terminal groups were reduced to primary amine groups using borane. Mn according to 1H NMR: 4500 g/mol.

### Example 8: [p(THF-EO)4000-U-C4H8-U]n

In a similar way as described in Example 1 for bis(3-aminopropyl)-poly(tetrahydrofuran) Mn=1100 g/mol, bis(3-aminopropyl)-poly(tetrahydrofuran-ran-ethylene oxide) Mn=4000 g/mol from Example 7 was reacted at room temperature with 1 molar equivalent of 1,4-diisocyanatobutane using chloroform as reaction solvent. After similar work-up as described in Example 1, the isolated polymer product was obtained as a white elastic fluffy material. The polymer product had a molecular weight of Mn = 58*103 g/mol as measured with SEC using NMP as eluent and relative to polystyrene standards. DSC: Tg = -73 °C, Tm1 = 1°C, Tm2 = 48 °C. T-flow = 140 °C.

### Example 9: [pCL2000-Urethane-Urea]n

Poly(e-caprolactone) diol (10 g, 5 mmol, Mn=2000 g/mol) was dissolved in 100 mL of CHCl3 dried over MgSO4 and filtered during transfer to the reaction flask. Under an argon atmosphere, 1,4-diisocyanatobutane (1.9 mL, 15 mmol) and 4 drops of dibutyltin dilaurate were added to this solution. This solution was refluxed overnight at 85°C under argon. After precipitation in heptane a white powder in a yield of 80% was obtained. This isocyanate-functionalized polycaprolactone (9.7 g, 4.2 mmol) was then dissolved in 200 mL dry CHCl3. Subsequently 1,4-diaminobutane (0.42 mL, 4.2 mmol) was dissolved in 50 mL dry CHCl3 and slowly added drop wise to the first solution until the isocyanate signal in FT-IR had disappeared. Precipitation in hexane resulted in a white flaky solid in 75% overall yield.

FT-IR: n = 3326, 2943, 2866, 1723, 1680, 1623, 1575, 1538 cm-1. 1H-NMR (CDCl3/MeOD): d = 5.2-5.0 (b, 6H), 4.23 (t, 4H), 4.06 (t, 2(2n)H), 3.70 (t, 4H), 3.16 (b, 12H), 2.31 (t, 2(2n)H), 1.65 (m, 2(4n)H), 1.51 (m, 12H), 1.40 (m, 2(2n)H) ppm, with n » 17. 13C-NMR (CDCl3) : d = 173.5, 68.7, 63.9, 63.0, 40.0, 39.7, 33.7, 28.3, 26.9, 26.7, 27.9, 25.1, 24.2 ppm. SEC: Mn= 86 kg/mole, Mw= 192 kg/mole, PDI= 2.2. DSC: Tg = -50 °C, Tm = 42 °C.

### Example 10: [pCL1250-Urethane-Urea]n

This polymer was synthesized in a manner similar to that used for [pCL2000-Urethane-Urea]n, except that polycaprolactone of Mn = 1250 g/mol was used as starting material. Overall yield = 56%, FT-IR, 1H-NMR (CDCl3/MeOD) and 13C-NMR (CDCl3) similar to that of [pCL2000-Urethane-Urea]n. DSC: Tg = -53 °C, Tm = 9°C.

### Example 11: [pCL2000-U-C4H8-U]n

Poly(e-caprolactone) diol (Mn=2000, 10 g, 5 mmol), N-carbobenzoxy-6-aminohexanoic acid (2.8 g, 11 mmol), 4-(dimethylamino)pyridinium 4-toluenesulfonate (DPTS) (0.7 g, 2.5 mmol) and DCC (3 g, 15 mmol) were dissolved in CHCl3 and the reaction was allowed to stir for 48 hours. The reaction mixture was filtered and the solvent was evaporated. The remaining solid material was dissolved in 100 mL CHCl3 and precipitated in hexane. To remove the remaining DPTS, the solid product was stirred in MeOH. After removing the MeOH, pCL2000 modified with N-carbobenzoxy groups was obtained as a white powder in a 64% yield. A solution of this polymer (4 g, 1.6 mmol) in 100 mL EtOAc/MeOH (v/v 2:1) and 400 mg of 10% Pd supported on activated carbon was subjected to hydrogenation under a H2 blanket at room temperature for 4 hours. After filtration over Celite, the product was isolated after precipitation in hexane as a white powder in a 95% yield. This intermediate product, pCL2000 modified with primary amine groups (14 g, 6.35 mmol), was dissolved in 100 mL CHCl3. A solution of 560 µL 1,4-diisocyanatobutane in 5 mL CHCl3 was slowly added by drops until the signal corresponding to amino methylene protons were no longer visible in 1H-NMR. The product was isolated in a 58% overall yield by precipitation in hexane. FT-IR: n = 3332, 2942, 2866, 1723, 1620, 1575 cm-1. 1H-NMR (CDCl3): d = 5.0-4.8 (b, 4H), 4.23 (t, 4H), 4.07 (t, 2(2n)H), 3.70 (t, 4H), 3.18 (b, 8H), 2.31 (t, 2(2n+2)H), 1.68 (m, 2(4n+2)H), 1.53 (m, 8H), 1.37 (m, 2(2n+2)H) ppm, with n » 17. 13C-NMR (CDCl3) : d = 173.5, 158.8, 69.0), 64.1, 63.2, 40.1, 39.8, 34.1, 29.9, 28.3, 27.5, 26.3, 25.5, 24.5 ppm. SEC: Mn= 34 kg/mole, Mw= 102 kg/mole, PD= 3.0. DSC: Tg = -54 °C, Tm1 = 27 °C, Tm2 = 98 °C.

### Example 12: [pCL1250-U-C4H8-U]n

This polymer was synthesized in a manner similar to that used for [pCL2000-U-C4H8-U]n. Overall yield = 31%, FT-IR, 1H-NMR (CDCl3/MeOD) and 13C-NMR (CDCl3) similar to that of [pCL2000-U-C4H8-U]n. SEC: Mn= 56 kg/mole, Mw= 109 kg/mole, PD= 1.9. DSC: Tg = -55 °C, Tm1 = 19 °C, Tm2 = 103 °C.

### Example 13: [pEthylene-Butylene3500- Urethane-Urea]n

Hydroxy-terminated poly(ethylene-ran-butylene) (hydrogenated polybutadiene, Kraton liquid polymer L-2203) (11.55 g) in 20 ml of CHCl3 was added drop wise over a period of two hours to a solution of isophorone diisocyanate (IPDI, 1.5 g) and a few drops of dibutyl tin laurate in 5 mL of CHCl3. The solution was stirred overnight under an argon atmosphere. Then, the solution was heated to 60 °C and was stirred for 2 hours. The mixture was cooled again to room temperature and 1,4-butyldiamine (0.3 g) in 3 ml of CHCl3 was added drop wise. The mixture was stirred overnight, after which completion of the reaction was confirmed by FT-IR analysis (no or hardly any isocyanate resonance peak was present). The material was isolated by precipitation into methanol, and subsequent drying. The material is highly elastic.

### Example 14: Mechanical properties as measured by tensile testing

Stress-strain measurements (tensile tests) were performed on a Zwick Z010 Universal Tensile Tester equipped with a 2.5 kN load cell at an elongation rate of 100% per minute. Tensile bars were punched from a solution-cast film of the polymers. The films of the polycaprolactone polymers from Examples 9-12 were thermally annealed at 80 oC or 100 °C. Typical dimensions of the tensile bars: length=22 mm, width=5.0 mm, and thickness=0.30 mm. Due to the shape of the curves, yield stresses were determined by determining the intersection point of the two tangents to the initial and final parts of the load elongation curves. An indicative Young's modulus was determined by calculating the slope at zero strain. The following Table shows the tensile testing data as recorded for the given polymer materials.

| Example | Polymer | Young's Strain modulus | Yield stress | Strength | at break | Toughness |
|---|---|---|---|---|---|---|
| | | E(MPa) | σ_{y} (MPa) | σ_{br} (MPa) | λ_{br} (%) | (kJ/kg) |
| **1** | **THF** | 96 | 9.6 | 28 | 1060 | 178 |
| **6** | **pTHF₂₀₀₀-U₂** | 26 | 4.7 | 28 | 1175 | 147 |
| **8** | **p(THF-EO)₄₀₀₀-U₂** | 11 | 2.4 | 11 | 2140 | 122 |
| **9** | **pCL₂₀₀₀-Ur-U₂** | 16 | 2.6 | 30 | 1114 | n.d. |
| **10** | **pCL₁₂₅₀-Ur-U₂** | 39 | 8.7 | 18 | 700 | n.d. |
| **11** | **pCL-₂₀₀₀-U₂** | 14 | 2.6 | 16 | 1330 | n.d. |
| **12** | **pCL₁₂₅₀-U₂** | 11 | 3.0 | 21 | 1505 | n.d. |

In DMTA-analyis (1Hz, 1 °C/min heating rate), the polymers of Examples 1, 6 and 8 show rubber plateaus at E' = 135 MPa, 17 MPa and 11 MPa, respectively. Flow is achieved at 148 °C, 112 °C and 105 °C, respectively.

### Example 15: The synthesis of aza-dyes 15A and 15B

### 4-Isocyanato-4'-nitroazobenzene.

Disperse Orange 3 (4-(4-Nitro-phenylazo)-aniline) (0.50 g, 2.07 mmol) was dissolved in THF (40 ml), and phosgene (2.2 ml 20% in toluene, 4.1 mmol) was added. The reaction mixture was heated to reflux temperature and stirred for 1 h, while argon was bubbled through the solution. It was evaporated to dryness, and the product was obtained as a red solid (0.62 g, 112%). FT-IR (ATR): n 2257 (NCO), 1734 (NHCOCl). cm-1.

### Aza-dye 15A: 3-(2-ethyl-hexyl)-1-[4-(4-nitro-phenylazo)-phenyl]-urea

4-Isocyanato-4'-nitroazobenzene (0.31 g, 1.00 mmol) was dissolved in THF (15 ml), and 2-ethylhexylamine (0.20 g, 1.5 mmol) in THF (5 ml) was added. The reaction mixture was stirred at room temperature for 30 min, after which it was evaporated to dryness. The product was redissolved in chloroform (20 ml), and extracted with hydrochloric acid solution (10 ml 0.1 M in water), and saturated sodium bicarbonate solution (10 ml). The organic layer was dried with sodium sulfate, filtered and purified by column chromatography using 1% methanol in chloroform as the eluent (Rf= 0.4 ). The product was obtained as an orange solid (0.30 g, 75%). 1H-NMR (DMSO-d6): d 9.02 (s, 1H, Ph-NH), 8.41 (d, 2H, C2'H, J=9.2 Hz), 8.01 (d, 2H, C3'H, J=8.8 Hz), 7.91 (d, 2H, C2H, J=8.8 Hz), 7.65 (d, 2H, C3H, J=8.8 Hz), 6.35 (t, 1H, CH2NH), 3.08 (q, 2H, CH2NH, J=5.9 Hz), 1.40 (m, 1H, CH), 1.28 (m, 8H, C-CH2-C), 0.89 (t, 6H, CH3, J=6.2 Hz). FT-IR (ATR): n 3336 (N-H stretching), 2960, 2928, 1669 (C=O stretching), 1595, 1543, 1515, 1340, 1226, 1140, 1105, 859, 843, 685 cm-1.

### Aza-dye 15B: 3-(2-ethyl-hexyl)-1-(3-[4-(4-nitro-phenylazo)-phenyl]-ureido-1,4-butyl)-urea

4-Isocyanato-4'-nitroazobenzene (0.55 g, 2.07 mmol) was dissolved in THF (30 ml), and 4-(tert-butoxycarbonylamino)-1-butylamine (0.58 g, 3.11 mmol) in THF (4 ml) was added. The reaction mixture was stirred at room temperature for 30 min, after which it was partially concentrated and precipitated in pentane (100 ml). The product was filtered off, and purified by column chromatography using 1% methanol in chloroform as the eluent (Rf=0.3). It was redissolved in dichloromethane (3 ml), and trifluoroacetic acid (2 ml) was added to deprotect the protected amine group. The reaction mixture was stirred at room temperature overnight, and subsequently evaporated to dryness to generate the aza-amine.

Di-tert-butyl tricarbonate (0.40 1.54 mmol) was dissolved in chloroform (10 ml), and 2-ethylhexyl amine (0.19 g, 1.47 mmol) in chloroform (2 ml) was injected into the former solution. The reaction mixture was stirred for 30 min to generate a solution of 2-ethyl hexyl isocyanate. The aza-amine was dissolved in pyridine (50 ml), and was added to the solution of 2-ethyl hexyl isocyanate. The reaction mixture was stirred for 30 min at room temperature, and then evaporated to dryness. The product was purified by column chromatography, first using pure chloroform as the eluent, than chloroform-methanol mixtures with up to 10% methanol (Rf= 0.2). The product was obtained as an orange solid (0.28 g, 27%). 1H-NMR (10% methanol-d4 in CDCl3): d 8.37 (d, 2H, C2'H, J=8.1 Hz), 7.99 (d, 2H, C3'H, J=8.4 Hz), 7.93 (d, 2H, C2H, J=8.4 Hz), 7.59 (d, 2H, C3H, J=9.2 Hz), 3.26 (t, 2H, PhNHCONHCH2), 3.15 (t, 2H, PhNHCONHCH2CH2CH2CH2), 3.07 (t, 2H, NHCONHCH2CH), 1.54 (m, 4H, NHCH2CH2CH2CH2NH), 1.4-1.2 (m, 9H, CH + CH2), 0.88 (t, 6H, CH3). FT-IR (ATR): n 3322 (N-H stretching), 2924, 2859, 1633 + 1623 (C=O stretching), 1584, 1552, 1523, 1343, 1226, 1140, 1106, 865, 754 cm-1. UV-Vis (THF): lmax=405 nm.

### Example 16: Incorporation of aza-dyes 15A and 15B into the polymer material of Example 1

### Preparation of aza-dye filled films

[pTHF1100-U-C4H8-U]n (ca. 2 g) and ca. 3 w/w% of aza-dye 15A or 15B were dissolved in chloroform (15 ml) and methanol (5 ml). These solutions were cast in silylated Petri-dishes (diameter 9 cm), and the solvent was allowed to evaporate slowly by placing a beaker over the dishes. After 20 h, the film was dried in vacuo at 50 °C for 5 h, and it was peeled off the Petri-dish. Both films containing either aza-dye 15A or aza-dye 15B were elastic, red and transparent. Microphase separation was not observed with optical microscopy for neither of the two films.

### Washing of the aza-dye filled films with a 0.1 M sodium dodecylsulphate (SDS) solution

Square pieces of 1 cm2 of the prepared red films were cut and they were individually stirred in a 0.1 M sodium dodecylsulfate (SDS) solution at 60°C for 90 minutes. This washing procedure had a remarkably different effect on the two pieces of polymer film. The film containing the aza-dye 15A that only has one urea group discoloured rapidly. After 90 minutes, it had become pale, while the washing water had an intense red colour, indicating that the aza-dye 15A is easily solubilized because it is loosely bound in the polymer material. In contrast, the piece of film containing aza-dye 15B kept its red colour. Even after prolonged washing, the washing water remained colourless, although the aza-dye 15B itself is readily soluble in the aqueous SDS-solution.

This experiment proves that the latter dye 15B is strongly anchored in the polymer material, whereas the aza-dye 15A is not, and thus can be easily washed out. The result can be explained by the fact that the aza-dye 15B and polymer [pTHF 1100-U-C4H8-U]n both contain bis-ureido-butylene units. This unit self-assembles, whereby the aza-dye 15B becomes strongly anchored into the polymer material.

### Example 17: Synthesis of a RGD-sequence containing peptide that also contains a bis-ureido-butylene unit

**1-Hexyl-3-(4-isocyanato-butyl)-urea**: Diisocyanatobutane (5.5 g, 39.5 mmol) was dissolved in 30 mL of dry chloroform and a solution of hexylamine (0.4 g, 3.95 mmol) in 10 mL of dry chloroform was added drop wise. The reaction was allowed to stir for 30 minutes after which the reaction mixture was filtered, the filtrate was reduced in volume and precipitated twice in hexane. A white solid was obtained in quantitative yield. FT-IR: 3325, 2955, 2930, 2860, 2264, 1614, 1571 cm-1. 1H-NMR (CDCl3): δ = 4.23 (b, 2H), 3.35 (t, 2H), 3.21 (t, 2H), 3.16 (t, 2H), 1.63, 1.49 and 1.29 (m, 12H), 0.89 (t, 3H).

**The peptide: (S)-N-((S)-1-Carboxy-2-hydroxy-ethyl)-3-(2-{(S)-5-guanidino-2-[2-(6-{3-[4-(3-hexyl-ureido)-butyl]-ureido}-hexanoylamino)-acetylamino]-pentanoylamino}-acetylamino)-succinamic acid.** Starting with the Wang-resin loaded with FMOC protected serine (1.5 g, 0.95 mmol), manual peptide chain assembly was carried out using DIPCDI/HOBt mediated (3.3/3.6 eq. with respect to peptide-resin) couplings in DMF. The Wang-resin loaded with FMOC protected serine was allowed to swell in DMF and the FMOC removal was achieved with 20% piperidine/DMF for 30 minutes followed by washes with DMF (3 ' 5 min). Three eq. of FMOC protected aminoacids were incorporated in separate syntheses; FMOC-Asp(OtBu) (1.2 g, 2.9 mmol), FMOC-Gly (0.84 g, 2.8 mmol), FMOC-Arg(PMC) (1.9 g, 2.9 mol) and FMOC-Gly (0.84 g, 2.8 mmol) were separately dissolved in DIPCDI/HOBt coupling reagents (6.5 ml) and were allowed to react at least 30 minutes with the loaded Wang-resin. Kaisertests, based on ninhydrin, showed the presence of free amine groups after each step, indicating a successful reaction (removal of FMOC or coupling of an aminoacid). The obtained product on the resin was washed with dichloromethane (2 ' 5 min) and with Et2O (1' 5 min) and dried by air. FMOC removal of this FMOC-GRGDS-resin (0.63 g, 0.26 mmol) was achieved with 20% piperidine/DMF and the GRGDS-resin was washed with DMF (3 ' 5 min) and allowed to swell. 6-(Fmoc-amino)caproic acid (0.32 g, 0.91 mmol) dissolved in 2.1 ml DMF containing DIPCDI/HOBt (1:1:1 eq.) was allowed to react with GRGDS-resin for one hour and was then washed with DMF (3 ' 5 min). FMOC was again removed by 20% piperidine/DMF. Three eq. 1-hexyl-3-(4-isocyanato-butyl)-urea (0.10 g, 0.43 mmol), were added and allowed to react overnight. After filtration, the resin was washed three times with DMF and three times with DCM.

The product was cleaved off the resin by 95% TFA/H2O (2 ml) at ambient conditions for six hours, filtered, precipitated in Et2O and spun down (2 minutes at 4300 RPM). The product was stirred up in Et2O and spun down three more times. The white residue was subsequently freeze dried three times from water with 10-33% acetonitrile, which resulted in a white fluffy powder. No TFA was observed anymore by 19F-NMR. LC-MS revealed one peak in the chromatogram with m/z observed mass: [M + H]+ = 845.5 g/mol and [M + H]2+ = 423.3 g/mol. Calculated mass: 844.96 g/mol.

### Example 18: Incorporation of the peptide of Example 17 into the polymer material of Example 11

The peptide of Example 17 was incorporated into the polymer material of Example 11 and, for reference, into polycaprolactone of molecular weight 80.000. This was done by dissolving the peptide and the polymer into a THF-solution, dropcasting this solution and let the THF evaporate. In both cases, 4 mol% of peptide was used, based on the amount of bis-urea units (i.e. the bis-ureido butylene units) in the components.

Both polymer samples were incubated with water at 37 °C during 48 hrs. In the case of the pCL80.000 material, 49% of the peptide was extracted out of the material into the water, whereas in the case of the [pCL2000-U-C4H8-U]n material of Example 11 only 26% of the peptide got extracted, implying that 74% of the peptide remained in this material. The percentages were determined using reversed phase liquid chromatography using mass spectrometry as detection (RPLC-MS).

The result shows that the peptide is anchored into the polymer material of Example 11, presumbaly because there is recognition between the bis-urea units present in both the polymer and the peptide component. The anchored peptide can be used to stimulate cell binding onto the polymer material, as the RGD-sequence is known to promote cell binding.

### Example 19: The biocompatibility of the polymer material of Example 11

In a cell proliferation assay, the proliferation of 3T3 mouse fibroblasts on the material [pCL2000-U-C4H8-U]n was compared to cell proliferation on cell culture polystyrene (PS), a known biocompatible material. Cells were seeded at a density of 1×103 or 1×104 cells/cm2 in duplicate. Cell proliferation was evaluated by optical microscopy on day 1, 3, 4 and 7. In all experiments, similar behavior was observed for cells seeded on [pCL2000-U-C4H8-U]n as compared to cells seeded on PS, demonstrating the biocompatibility of the material of Example 11.

In a second in vitro biocompatibility test, the cell viability of 3T3 mouse fibroblasts seeded in medium that was incubated with [pCL2000-U-C4H8-U]n, UHMWPE (ultra high molecular weight polyethylene) or latex was investigated using a LDH (lactate dehydrogenase) test. Every 24 hours the medium was refreshed and all collected medium was used in a LDH activity assay. UHMWPE is known to be biocompatible while latex is not, and this was also found here: cell viabilities exceeding 90% and below 5% were found for UHMWPE and latex, respectively. The cell viability for [pCL2000-U-C4H8-U]n was only approximately 50% when no prewash was applied, but after two prewashes, the cell viabilities were exceeding 90% proving the biocompatibility of the polymer material. We attribute the initial lower cell viability to the presence of small amounts of remaining solvent.

## Claims

1. A prosthesis device comprising a body at least partly formed from a segmented thermoplastic elastomer having crystallized blocks and at least one functional component, which is able to chemically bond to the crystallized blocks and has cartilage regenerative properties, , wherein the crystallized blocks comprise bis-urea moieties.

2. Prosthesis device according to claim 1, wherein the segmented thermoplastic elastomer is a thermoplastic elastomeric polyurethane.

3. Prosthesis device according to claim 1 or 2, wherein the at least one functional component also bears the bis-urea moieties.

4. Prosthesis device according to any one of claims 1 - 3, whereby the amount of the functional component is lower than 20 mol% with respect to the total amount of bis-urea moieties in the elastomer.

5. Prosthesis device according to any one of claims 1 - 4, wherein the at least one functional component comprises a peptide.

6. Prosthesis device according to claim 5, wherein the peptide comprises at least one RGD-sequence.

7. A prosthesis device according to any one of the preceding claims, which body provides cushioning and load distribution capabilities through the cartilage, and is shaped such that it fits with a femoral condyle, a tubercle, and a tibial plateau, and stays within the joint space without any separate means of attachment.

8. Prosthesis device according to any one of the preceding claims, having a superior surface forming a concave groove channel contoured to receive a femoral condyle, and an inferior surface forming a convex surface contoured to fit on top a tibial plateau.

9. Prosthesis device according to any one of the preceding claims, wherein the body is substantially kidney shaped, toroidal in shape or crescent shaped.

10. Prosthesis device according to any one of the preceding claims, wherein the body is attached to a tissue fixation component.

11. Prosthesis device according to claim 10, wherein the tissue fixation component is selected from the group consisting of extension tabs, sutures, and mesh.

12. A prosthesis device according to any one of the preceding claims, which body further comprises a reinforcing material selected from the group consisting of polymers and/or metals.

13. Prosthesis device according to claim 12, wherein the reinforcing material is a foam.

14. Prosthesis device according to claim 13, wherein the foam forms the core of the body and the elastomer the skin.

## Patentansprüche

1. Protheseeinheit, die einen Körper umfasst, der mindestens teilweise aus einem segmentierten thermoplastischen Elastomer gebildet wird, das kristallisierte Blöcke und mindestens ein funktionales Element aufweist, das sich chemisch mit den kristallisierten Blöcken verbinden kann und knorpelregenerative Eigenschaften hat, wobei die kristallisierten Blöcke Bisharnstoffanteile umfassen.

2. Protheseeinheit nach Anspruch 1, wobei das segmentierte thermoplastische Elastomer ein thermoplastisches Elastomer-Polyurethan ist.

3. Protheseeinheit nach Anspruch 1 oder 2, wobei das mindestens eine funktionale Element auch die Bisharnstoffanteile umfasst.

4. Protheseeinheit nach einem der Ansprüche 1 bis 3, wobei die Menge des funktionalen Elements weniger als 20 Mol% in Bezug auf die Gesamtmenge der Bisharnstoffanteile in dem Elastomer beträgt.

5. Protheseeinheit nach einem der Ansprüche 1 bis 4, wobei das mindestens eine funktionale Element ein Peptid umfasst.

6. Protheseeinheit nach Anspruch 5, wobei das Peptid mindestens eine RGD-Sequenz umfasst.

7. Protheseeinheit nach einem der oben genannten Ansprüche, wobei der Körper Polsterung und Lastverteilungsfähigkeiten durch den Knorpel bereitstellt und so geformt ist, dass er für einen Femurkondylus, einen Tuberkel und einen Schienbeinkopf geeignet ist und ohne separate Befestigungsmittel im Gelenkraum verbleibt.

8. Protheseeinheit nach einem der oben genannten Ansprüche, die eine obere Fläche aufweist, welche einen konkaven Rillenkanal bildet, der gestaltet ist, um einen Femurkondylus aufzunehmen, sowie eine untere Fläche, die eine konvexe Fläche bildet, die gestaltet ist, um auf einen Schienbeinkopf zu passen.

9. Protheseeinheit nach einem der oben genannten Ansprüche, wobei der Körper im Wesentlichen nierenförmig, ringförmig oder sichelförmig ist.

10. Protheseeinheit nach einem der oben genannten Ansprüche, wobei der Körper an einem Gewebebefestigungselement angebracht ist.

11. Protheseeinheit nach Anspruch 10, wobei das Gewebebefestigungselement aus der Gruppe ausgewählt wird, die aus Verlängerungsstreifen, Nähten und Maschengeflecht besteht.

12. Protheseeinheit nach einem der oben genannten Ansprüche, wobei der Körper weiterhin ein Verstärkungsmaterial umfasst, das aus der Gruppe ausgewählt wird, die aus Polymeren und/oder Metallen besteht.

13. Protheseeinheit nach Anspruch 12, wobei das Verstärkungsmaterial ein Schaum ist.

14. Protheseeinheit nach Anspruch 13, wobei der Schaum den Kern des Körpers und das Elastomer die Außenhaut bildet.

## Revendications

1. Dispositif prothétique comprenant un corps au moins partiellement fait en un élastomère thermoplastique segmenté comportant des séquences cristallisées et au moins un composant fonctionnel qui est capable de se lier chimiquement aux séquences cristallisées et possède des propriétés de régénération du cartilage, étant entendu que les séquences cristallisées sont constituées de bis-urée.

2. Dispositif prothétique selon la revendication 1, dans lequel l'élastomère thermoplastique segmenté est un polyuréthane élastomère thermoplastique.

3. Dispositif prothétique selon la revendication 1 ou 2, dans lequel le au moins un composant fonctionnel porte également les fragments de bis-urée.

4. Dispositif prothétique selon l'une quelconque des revendications 1-3, la quantité du composant fonctionnel étant inférieure à 20 % en mole par rapport à la quantité totale de fragments de bis-urée dans l'élastomère.

5. Dispositif prothétique selon l'une quelconque des revendications 1-4, dans lequel le au moins un composant fonctionnel est constitué d'un peptide.

6. Dispositif prothétique selon la revendication 5, dans lequel le peptide comprend au moins une séquence RGD.

7. Dispositif prothétique selon l'une quelconque des revendications précédentes, lequel corps procure des capacités d'amortissement et de distribution des charges dans le cartilage et est conformé de telle sorte qu'il s'adapte à un condyle fémoral, une tubérosité et un plateau tibial, et reste dans l'interligne articulaire sans aucun moyen séparé de fixation.

8. Dispositif prothétique selon l'une quelconque des revendications précédentes, comportant une surface supérieure formant un canal formant rainure concave configuré pour recevoir un condyle fémoral, et une surface inférieure formant une surface convexe configurée pour s'appliquer par-dessus un plateau tibial.

9. Dispositif prothétique selon l'une quelconque des revendications précédentes, dans lequel le corps est sensiblement réniforme, de forme toroïdale ou en forme de croissant.

10. Dispositif prothétique selon l'une quelconque des revendications précédentes, dans lequel le corps est attaché à un composant de fixation de tissus.

11. Dispositif prothétique selon la revendication 10, dans lequel le composant de fixation de tissus est sélectionné dans le groupe constitué de languettes d'extension, de sutures et de treillis.

12. Dispositif prothétique selon l'une quelconque des revendications précédentes, dans lequel le corps comprend par ailleurs un matériau de renfort sélectionné dans le groupe constitué de polymères et/ou de métaux.

13. Dispositif prothétique selon la revendication 12, dans lequel le matériau de renfort est une mousse.

14. Dispositif prothétique selon la revendication 13, dans lequel la mousse forme l'âme du corps et l'élastomère, la peau.
